# EUROPEAN PATENT APPLICATION

(11) **EP 2 527 443 A2**
(43) Date of publication of application: **28.11.2012**
(21) Application number: 12174878.4
(22) Date of filing: 29.12.2006
(51) Int. Cl.: C12N 15/113

(54) **RNAi-mediated inhibition of IGF-1R for treatment of ocular angiogenesis**

(30) Priority: 29.12.2005 US 754796 P
(62) Divisional of application: 06840364.1
(71) Applicant: ALCON RESEARCH, LTD., 76134-2099 TX, Texas Fort Worth (US)
(72) Inventor: Chatterton, Jon E., Crowley, TX 76036 (US); Bingaman, David P., Weatherford, TX 76088 (US)
(74) Representative: Keller, Günter

(57) **Abstract**

RNA interference is provided for inhibition of IGF1R mRNA expression for treating patients with ocular angiogenesis, particularly for treating retinal edema, diabetic retinopathy, sequela associated with retinal ischemia, posterior segment neovascularization (PSNV), and neovascular glaucoma, and for treating patients at risk of developing such conditions.

SEQUENCE LISTING

<110> Chatterton, Jon E.
      Bingaman, David P.

<120> RNAi-Mediated Inhibition of IGF1R For Treatment of Ocular
      Angiogenesis

<130> 45263-P017US

<150> 60/754,796
<151> 2005-12-29

<160> 45

<170> PatentIn version 3.3

<210> 1
<211> 4989
<212> DNA
<213> Homo sapiens

<400> 1
tttttttttt ttttgagaaa gggaatttca tcccaaataa aaggaatgaa gtctggctcc      60

ggaggagggt ccccgacctc gctgtggggg ctcctgtttc tctccgccgc gctctcgctc     120

tggccgacga gtggagaaat ctgcgggcca ggcatcgaca tccgcaacga ctatcagcag     180

ctgaagcgcc tggagaactg cacggtgatc gagggctacc tccacatcct gctcatctcc     240

aaggccgagg actaccgcag ctaccgcttc cccaagctca cggtcattac cgagtacttg     300

ctgctgttcc gagtggctgg cctcgagagc ctcggagacc tcttccccaa cctcacggtc     360

atccgcggct ggaaactctt ctacaactac gccctggtca tcttcgagat gaccaatctc     420

aaggatattg ggctttacaa cctgaggaac attactcggg gggccatcag gattgagaaa     480

aatgctgacc tctgttacct ctccactgtg gactggtccc tgatcctgga tgcggtgtcc     540

aataactaca ttgtggggaa taagccccca aaggaatgtg gggacctgtg tccagggacc     600

atggaggaga agccgatgtg tgagaagacc accatcaaca atgagtacaa ctaccgctgc     660

tggaccacaa accgctgcca gaaaatgtgc ccaagcacgt gtgggaagcg ggcgtgcacc     720

gagaacaatg agtgctgcca ccccgagtgc ctgggcagct gcagcgcgcc tgacaacgac     780

acggcctgtg tagcttgccg ccactactac tatgccggtg tctgtgtgcc tgcctgcccg     840

cccaacacct acaggtttga gggctggcgc tgtgtggacc gtgacttctg cgccaacatc     900

ctcagcgccg agagcagcga ctccgagggg tttgtgatcc acgacggcga gtgcatgcag     960

gagtgcccct cgggcttcat ccgcaacggc agccagagca tgtactgcat cccttgtgaa    1020

ggtccttgcc cgaaggtctg tgaggaagaa aagaaaacaa agaccattga ttctgttact    1080

tctgctcaga tgctccaagg atgcaccatc ttcaagggca atttgctcat taacatccga    1140

cgggggaata acattgcttc agagctggag aacttcatgg ggctcatcga ggtggtgacg    1200

ggctacgtga agatccgcca ttctcatgcc ttggtctcct tgtccttcct aaaaaacctt    1260

cgcctcatcc taggagagga gcagctagaa gggaattact ccttctacgt cctcgacaac 1320
cagaacttgc agcaactgtg ggactgggac caccgcaacc tgaccatcaa agcagggaaa 1380
atgtactttg ctttcaatcc caaattatgt gtttccgaaa tttaccgcat ggaggaagtg 1440
acggggacta aagggcgcca aagcaaaggg gacataaaca ccaggaacaa cggggagaga 1500
gcctcctgtg aaagtgacgt cctgcatttc acctccacca ccacgtcgaa gaatcgcatc 1560
atcataacct ggcaccggta ccggccccct gactacaggg atctcatcag cttcaccgtt 1620
tactacaagg aagcaccctt taagaatgtc acagagtatg atgggcagga tgcctgcggc 1680
tccaacagct ggaacatggt ggacgtggac ctcccgccca acaaggacgt ggagcccggc 1740
atcttactac atgggctgaa gccctggact cagtacgccg tttacgtcaa ggctgtgacc 1800
ctcaccatgg tggagaacga ccatatccgt ggggccaaga gtgagatctt gtacattcgc 1860
accaatgctt cagttccttc cattcccttg gacgttcttt cagcatcgaa ctcctcttct 1920
cagttaatcg tgaagtggaa ccctccctct ctgcccaacg gcaacctgag ttactacatt 1980
gtgcgctggc agcggcagcc tcaggacggc tacctttacc ggcacaatta ctgctccaaa 2040
gacaaaatcc ccatcaggaa gtatgccgac ggcaccatcg acattgagga ggtcacagag 2100
aaccccaaga ctgaggtgtg tggtggggag aaagggcctt gctgcgcctg ccccaaaact 2160
gaagccgaga agcaggccga gaaggaggag gctgaatacc gcaaagtctt tgagaatttc 2220
ctgcacaact ccatcttcgt gcccagacct gaaaggaagc ggagagatgt catgcaagtg 2280
gccaacacca ccatgtccag ccgaagcagg aacaccacgg ccgcagacac ctacaacatc 2340
accgacccgg aagagctgga gacagagtac cctttctttg agagcagagt ggataacaag 2400
gagagaactg tcatttctaa ccttcggcct ttcacattgt accgcatcga tatccacagc 2460
tgcaaccacg aggctgagaa gctgggctgc agcgcctcca acttcgtctt tgcaaggact 2520
atgcccgcag aaggagcaga tgacattcct gggccagtga cctgggagcc aaggcctgaa 2580
aactccatct ttttaaagtg gccggaacct gagaatccca atggattgat tctaatgtat 2640
gaaataaaat acggatcaca agttgaggat cagcgagaat gtgtgtccag acaggaatac 2700
aggaagtatg gaggggccaa gctaaaccgg ctaaacccgg ggaactacac agcccggatt 2760
caggccacat ctctctctgg gaatgggtcg tggacagatc ctgtgttctt ctatgtccag 2820
gccaaaacag gatatgaaaa cttcatccat ctgatcatcg ctctgcccgt cgctgtcctg 2880
ttgatcgtgg gagggttggt gattatgctg tacgtcttcc atagaaagag aaataacagc 2940
aggctgggga atggagtgct gtatgcctct gtgaacccgg agtacttcag cgctgctgat 3000
gtgtacgttc ctgatgagtg ggaggtggct cgggagaaga tcaccatgag ccgggaactt 3060
gggcaggggt cgtttgggat ggtctatgaa ggagttgcca agggtgtggt gaaagatgaa 3120
cctgaaacca gagtggccat taaaacagtg aacgaggccg caagcatgcg tgagaggatt 3180
gagtttctca acgaagcttc tgtgatgaag gagttcaatt gtcaccatgt ggtgcgattg 3240

ctgggtgtgg tgtcccaagg ccagccaaca ctggtcatca tggaactgat gacacggggc 3300

gatctcaaaa gttatctccg gtctctgagg ccagaaatgg agaataatcc agtcctagca 3360

cctccaagcc tgagcaagat gattcagatg gccggagaga ttgcagacgg catggcatac 3420

ctcaacgcca ataagttcgt ccacagagac cttgctgccc ggaattgcat ggtagccgaa 3480

gatttcacag tcaaaatcgg agattttggt atgacgcgag atatctatga gacagactat 3540

taccggaaag gaggcaaagg gctgctgccc gtgcgctgga tgtctcctga gtccctcaag 3600

gatggagtct tcaccactta ctcggacgtc tggtccttcg gggtcgtcct ctgggagatc 3660

gccacactgg ccgagcagcc ctaccagggc ttgtccaacg agcaagtcct tcgcttcgtc 3720

atggagggcg gccttctgga caagccagac aactgtcctg acatgctgtt tgaactgatg 3780

cgcatgtgct ggcagtataa ccccaagatg aggccttcct tcctggagat catcagcagc 3840

atcaaagagg agatggagcc tggcttccgg gaggtctcct tctactacag cgaggagaac 3900

aagctgcccg agccggagga gctggacctg gagccagaga acatggagag cgtccccctg 3960

gacccctcgg cctcctcgtc ctccctgcca ctgcccgaca gacactcagg acacaaggcc 4020

gagaacggcc ccggccctgg ggtgctggtc ctccgcgcca gcttcgacga gagacagcct 4080

tacgcccaca tgaacggggg ccgcaagaac gagcgggcct tgccgctgcc ccagtcttcg 4140

acctgctgat ccttggatcc tgaatctgtg caaacagtaa cgtgtgcgca cgcgcagcgg 4200

ggtggggggg gagagagagt tttaacaatc cattcacaag cctcctgtac ctcagtggat 4260

cttcagttct gcccttgctg cccgcgggag acagcttctc tgcagtaaaa cacatttggg 4320

atgttccttt tttcaatatg caagcagctt tttattccct gcccaaaccc ttaactgaca 4380

tgggccttta agaaccttaa tgacaacact taatagcaac agagcacttg agaaccagtc 4440

tcctcactct gtccctgtcc ttccctgttc tccctttctc tctcctctct gcttcataac 4500

ggaaaaataa ttgccacaag tccagctggg aagccctttt tatcagtttg aggaagtggc 4560

tgtccctgtg gccccatcca accactgtac acacccgcct gacaccgtgg gtcattacaa 4620

aaaaacacgt ggagatggaa atttttacct ttatctttca cctttctagg gacatgaaat 4680

ttacaaaggg ccatcgttca tccaaggctg ttaccatttt aacgctgcct aattttgcca 4740

aaatcctgaa ctttctccct catcggcccg gcgctgattc ctcgtgtccg gaggcatggg 4800

tgagcatggc agctggttgc tccatttgag agacacgctg gcgacacact ccgtccatcc 4860

gactgcccct gctgtgctgc tcaaggccac aggcacacag gtctcattgc ttctgactag 4920

attattattt gggggaactg gacacaatag gtctttctct cagtgaaggt ggggagaagc 4980

tgaaccggc 4989

<210> 2
<211> 19
<212> DNA

<213>  Artificial

<220>
<223>  Target Sequence

<400>  2
tcttcgagat gaccaatct                                                    19


<210>  3
<211>  21
<212>  DNA
<213>  Artificial

<220>
<223>  Sense strand with 3'NN


<220>
<221>  misc_RNA
<222>  (1)..(19)
<223>  Ribonucleotides

<220>
<221>  misc_feature
<222>  (20)..(21)
<223>  any, A, T/U, C, G

<400>  3
ucuucgagau gaccaaucun n                                                 21


<210>  4
<211>  21
<212>  DNA
<213>  Artificial

<220>
<223>  Antisense strand with 3'NN


<220>
<221>  misc_RNA
<222>  (1)..(19)
<223>  Ribonucleotides

<220>
<221>  misc_feature
<222>  (20)..(21)
<223>  any, A, T/U, C, G

<400>  4
agauugguca ucucgaagan n                                                 21


<210>  5
<211>  21
<212>  RNA
<213>  Artificial

<220>
<223>  Sense Strand

<400>  5
ucuucgagau gaccaaucuu u                                                 21

<210>  6
<211>  21
<212>  RNA
<213>  Artificial

<220>
<223>  Antisense strand

<400>  6
agauugguca ucucgaagau u                                                21


<210>  7
<211>  48
<212>  DNA
<213>  Artificial

<220>
<223>  Hairpin duplex with loop


<220>
<221>  misc_RNA
<222>  (1)..(19)
<223>  Ribonucleotides

<220>
<221>  misc_feature
<222>  (20)..(27)
<223>  any, A, T/U, C, G

<220>
<221>  misc_feature
<222>  (28)..(48)
<223>  Ribonucleotides

<400>  7
ucuucgagau gaccaaucun nnnnnnnaga uuggucaucu cgaagauu                   48


<210>  8
<211>  19
<212>  DNA
<213>  Artificial

<220>
<223>  Target Sequence

<400>  8
tcaacaatga gtacaacta                                                   19


<210>  9
<211>  19
<212>  DNA
<213>  Artificial

<220>
<223>  Target Sequence

<400>  9
gaccattgat tctgttact                                                   19

<210>  10
<211>  19
<212>  DNA
<213>  Artificial

<220>
<223>  Target Sequence

<400>  10
gaagaatcgc atcatcata                                                    19


<210>  11
<211>  19
<212>  DNA
<213>  Artificial

<220>
<223>  Target Sequence

<400>  11
tcatcagctt caccgttta                                                    19


<210>  12
<211>  19
<212>  DNA
<213>  Artificial

<220>
<223>  Target Sequence

<400>  12
agaatgtcac agagtatga                                                    19


<210>  13
<211>  19
<212>  DNA
<213>  Artificial

<220>
<223>  Target Sequence

<400>  13
ggactcagta cgccgttta                                                    19


<210>  14
<211>  19
<212>  DNA
<213>  Artificial

<220>
<223>  Target Sequence

<400>  14
agttaatcgt gaagtggaa                                                    19


<210>  15
<211>  19
<212>  DNA
<213>  Artificial

<220>
<223>  Target Sequence

<400>  15
acctttaccg gcacaatta                                                    19


<210>  16
<211>  19
<212>  DNA
<213>  Artificial

<220>
<223>  Target Sequence

<400>  16
acggcaccat cgacattga                                                    19


<210>  17
<211>  19
<212>  DNA
<213>  Artificial

<220>
<223>  Target Sequence

<400>  17
ttgagaattt cctgcacaa                                                    19


<210>  18
<211>  19
<212>  DNA
<213>  Artificial

<220>
<223>  Target Sequence

<400>  18
tctaaccttc ggcctttca                                                    19


<210>  19
<211>  19
<212>  DNA
<213>  Artificial

<220>
<223>  Target Sequence

<400>  19
ttcggccttt cacattgta                                                    19


<210>  20
<211>  19
<212>  DNA
<213>  Artificial

<220>
<223>  Target Sequence

<400>  20

gatcacaagt tgaggatca                                                    19

<210>  21
<211>  19
<212>  DNA
<213>  Artificial

<220>
<223>  Target Sequence

<400>  21
tgtacgtctt ccatagaaa                                                    19

<210>  22
<211>  19
<212>  DNA
<213>  Artificial

<220>
<223>  Target Sequence

<400>  22
ggagaataat ccagtccta                                                    19

<210>  23
<211>  19
<212>  DNA
<213>  Artificial

<220>
<223>  Target Sequence

<400>  23
catacctcaa cgccaataa                                                    19

<210>  24
<211>  19
<212>  DNA
<213>  Artificial

<220>
<223>  Target Sequence

<400>  24
attgcatggt agccgaaga                                                    19

<210>  25
<211>  19
<212>  DNA
<213>  Artificial

<220>
<223>  Target Sequence

<400>  25
ccgaagattt cacagtcaa                                                    19

<210>  26
<211>  19

<212>  DNA
<213>  Artificial

<220>
<223>  Target Sequence

<400>  26
tttggtatga cgcgagata                                                    19


<210>  27
<211>  19
<212>  DNA
<213>  Artificial

<220>
<223>  Target Sequence

<400>  27
tgacgcgaga tatctatga                                                    19


<210>  28
<211>  19
<212>  DNA
<213>  Artificial

<220>
<223>  Target Sequence

<400>  28
cgcatgtgct ggcagtata                                                    19


<210>  29
<211>  19
<212>  DNA
<213>  Artificial

<220>
<223>  Target Sequence

<400>  29
gcatgtgctg gcagtataa                                                    19


<210>  30
<211>  19
<212>  DNA
<213>  Artificial

<220>
<223>  Target Sequence

<400>  30
tctactacag cgaggagaa                                                    19


<210>  31
<211>  19
<212>  DNA
<213>  Artificial

<220>
<223>  Target Sequence

<400>  31
tcgacgagag acagcctta                                                    19


<210>  32
<211>  19
<212>  DNA
<213>  Artificial

<220>
<223>  Target Sequence

<400>  32
tcctgaatct gtgcaaaca                                                    19


<210>  33
<211>  19
<212>  DNA
<213>  Artificial

<220>
<223>  Target Sequence

<400>  33
taatagcaac agagcactt                                                    19


<210>  34
<211>  19
<212>  DNA
<213>  Artificial

<220>
<223>  Target Sequence

<400>  34
ctctgcttca taacggaaa                                                    19


<210>  35
<211>  19
<212>  DNA
<213>  Artificial

<220>
<223>  Target Sequence

<400>  35
tcattgcttc tgactagat                                                    19


<210>  36
<211>  19
<212>  DNA
<213>  Artificial

<220>
<223>  Target Sequence

<400>  36
cattgcttct gactagatt                                                    19

<210>  37
<211>  19
<212>  DNA
<213>  Artificial

<220>
<223>  Target Sequence

<400>  37
gcttctgact agattatta                                                    19


<210>  38
<211>  19
<212>  DNA
<213>  Artificial

<220>
<223>  Target Sequence

<400>  38
ggccagaaat ggagaataa                                                    19


<210>  39
<211>  19
<212>  DNA
<213>  Artificial

<220>
<223>  Target Sequence

<400>  39
gcagacacct acaacatca                                                    19


<210>  40
<211>  19
<212>  DNA
<213>  Artificial

<220>
<223>  Target Sequence

<400>  40
gtgggagggt tggtgatta                                                    19


<210>  41
<211>  19
<212>  RNA
<213>  Artificial

<220>
<223>  Sense strand

<400>  41
ucuucgagau gaccaaucu                                                    19


<210>  42
<211>  19
<212>  RNA
<213>  Artificial

<220>
<223>  Antisense strand

<400>  42
agauugguca ucucgaaga                                                    19


<210>  43
<211>  25
<212>  DNA
<213>  Artificial

<220>
<223>  Sense strand

<400>  43
tcttcgagat gaccaatctc aagga                                             25


<210>  44
<211>  25
<212>  RNA
<213>  Artificial

<220>
<223>  Sense Strand

<400>  44
ucuucgagau gaccaaucuc aagga                                             25


<210>  45
<211>  27
<212>  RNA
<213>  Artificial

<220>
<223>  Antisense strand

<400>  45
uccuugagau uggucaucuc gaagauu                                           27

## Description

The present application claims the benefit of co-pending U.S. Provisional Patent Application Serial Number 60/754,796 filed December 29, 2005, the text of which is specifically incorporated by reference herein.

### FIELD OF THE INVENTION

The present invention relates to the field of interfering RNA compositions for inhibition of expression of insulin-like growth factor-1 receptor (IGF-1R), the protein encoded by IGF1R mRNA, in ocular angiogenesis, including those cellular changes resulting from the interaction of insulin-like growth factor-1 (IGF-1) and IGF-1R that lead directly or indirectly to ocular neovascularization, retinal edema, diabetic retinopathy, sequela associated with retinal ischemia, posterior segment neovascularization, and neovascular glaucoma, for example.

### BACKGROUND OF THE INVENTION

Diabetic retinopathy (DR) is an eye disease that develops in diabetes due to changes in the cells that line blood vessels, i.e. the retinal microvascular endothelium. During diabetes mellitus, hyperglycemia can cause damage in a number of ways. For example, glucose, or a metabolite of glucose, binds to the amino groups of proteins, leading to tissue damage. In addition, excess glucose enters the polyol pathway resulting in accumulations of sorbitol. Sorbitol cannot be metabolized by the cells of the retina and can contribute to high intracellular osmotic pressure, intracellular edema, impaired diffusion, tissue hypoxia, capillary cell damage, and capillary weakening. Diabetic retinopathy involves thickening of capillary basement membranes which may in turn prevent pericytes, the predominant perivascular cell type in retinal capillaries, from contacting endothelial cells. Pericyte and endothelial cell death occurs through an apoptotic mechanism during diabetic retinopathy, where the loss of pericytes likely increases the permeability of the capillaries and leads to breakdown of the blood-retina barrier and blood flow dysregulation. Weakened capillaries lead to aneurysm formation and further leakage. These effects of hyperglycemia can also impair neuronal functions in the retina. DR is associated with retinal microaneurysms, hemorrhages, exudates, and retinitis proliferans, i.e., massive neovascular and connective tissue growth on the inner surface of the retina. Diabetic retinopathy may be of the background type, progressively characterized by microaneurysms; intraretinal punctate hemorrhages; yellow, waxy exudates; cotton-wool patches; and macular edema. This is an early stage of diabetic retinopathy termed nonproliferative diabetic retinopathy.

As the diabetes-induced microvascular pathology progress, retinal capillaries eventually become occluded and lead to multifocal areas of ischemia hypoxia within the retina. Hypoxic conditions in the non-perfused tissue elicits the production of growth factors capable of stimulating abnormal new blood vessel growth from existing vessels (angiogenesis). These pathologic new blood vessels grow into the vitreous and can cause loss of sight, a condition called proliferative diabetic retinopathy (PDR), since the new blood vessels are fragile and tend to leak blood into the eye. The proliferative type of DR is characterized by neovascularization of the retina and optic disk which may project into the vitreous, proliferation of fibrous tissue, vitreous hemorrhage, and retinal detachment.

Neovascularization also occurs in a type of glaucoma called neovascular glaucoma in which increased intraocular pressure is caused by growth of connective tissue and new blood vessels upon the trabecular meshwork. Neovascular glaucoma is a form of secondary glaucoma caused by neovascularization in the chamber angle.

Posterior segment neovascularization (PSNV) is a vision-threatening pathology responsible for the two most common causes of acquired blindness in developed countries: exudative age-related macular degeneration (AMD) and PDR. Until recently, the only approved treatments for PSNV that occurs during exudative AMD were laser photocoagulation or photodynamic therapy with VISUDYNE™. Both therapies involve occlusion of affected vasculature, which results in permanent, laser-induced damage to the retina, and does not address the underlying cause of neovascularization. Recurrence of neovascularization from the same area is common. For patients with PDR, surgical interventions with vitrectomy and removal of preretinal membranes are the only options currently available, as well as a laser therapy called panretinal photocoagulation to prevent the production of more new vessels.

Current pharmaceutical efforts have focused on inhibiting the effects of potent angiogenic factors such as VEGF. Recently, intravitreal injection of LUCENTIS™, an anti-VEGF antibody fragment, was approved for treatment of AMD. This antibody fragment was designed to bind to and inhibit VEGF to inhibit the formation of new blood vessels. Lucentis is also in clinical trials for the treatment of diabetic macular edema. Other approaches include the use of small interfering RNA targeting VEGF or its receptor.

The growth hormone (GH)/IGF1 axis is implicated in DR as evidenced by results showing an increase in IGF1 in ocular fluids and tissues from patients with advancing DR. Further, patients treated subcutaneously with octreotide, a somatostatin analog that inhibits the GH/IGF1 axis, show empirical improvement in diabetic macular edema and PDR. In the mouse OIR model, treatment with a GH inhibitor or IGF1R antagonist significantly decreases retinal neovascularization. In a mouse diabetic model, plasmid-mediated IGF-1 therapy reversed diabetic increased angiogenesis and arterial flow.

IGF1R is a member of the receptor tyrosine kinase family. Several small molecule receptor tyrosine kinase inhibitors (RTKi) have been described that inhibit retinal neovascularization and/or choroidal neovascularization in mice. Each of these molecules inhibits multiple kinases which may be effective in blocking neovascularization, however, each has the attendant risk of causing toxic side effects. A small molecule drug that will inhibit all of the kinases necessary to block neovascularization may also inhibit a kinase that is needed for cell survival.

The present invention addresses this lack of specificity of inhibition of receptor tyrosine kinases, specifically the insulin-like growth factor-1 receptor. The present invention provides interfering RNAs targeting IGF1R in angiogenesis and vascular permeability.

### SUMMARY OF THE INVENTION

The present invention is directed to interfering RNAs that silence IGF1R mRNA expression, thus decreasing activity of the IGF-1/IGF-1R bound complex and treating ocular angiogenesis by effecting a lowering of ocular pre-angiogenic and angiogenic cellular activity. IGF-1R is activated by the binding of IGF-1 to the extracellular domain of the receptor. The activation of the kinase in turn results in the stimulation of different intracellular substrates.

The term "ocular angiogenesis," as used herein, includes ocular pre-angiogenic conditions and ocular angiogenic conditions, and includes those cellular changes resulting from the interaction of IGF-1 and IGF-1R that lead directly or indirectly to ocular angiogenesis, ocular neovascularization, retinal edema, diabetic retinopathy, sequela associated with retinal ischemia, PSNV, vascular permeability, and neovascular glaucoma, for example. The interfering RNAs of the invention are useful for treating patients with ocular angiogenesis, ocular neovascularization, retinal edema, diabetic retinopathy, sequela associated with retinal ischemia, posterior segment neovascularization (PSNV), and neovascular glaucoma, or patients at risk of developing such conditions, for example.

An embodiment of the present invention provides a method of attenuating expression of an IGF1R mRNA target in a subject. The method comprises administering to the subject a composition comprising an effective amount of interfering RNA such as an siRNA having a length of 19 to 49 nucleotides and a pharmaceutically acceptable carrier. Administration is to an eye of the subject for attenuating expression of an ocular angiogenesis target in a human.

In one embodiment of the invention, the interfering RNA comprises a sense nucleotide strand, an antisense nucleotide strand and a region of at least near-perfect contiguous complementarity of at least 19 nucleotides. Further, the antisense strand hybridizes under physiological conditions to a portion of an mRNA corresponding to SEQ ID NO:1 which is the sense cDNA sequence encoding IGF1R (GenBank accession no. NM_000875) and has a region of at least near-perfect contiguous complementarity of at least 19 nucleotides with the hybridizing portion of mRNA corresponding to SEQ ID NO:1. The administration of such a composition attenuates the expression of an IGF1R mRNA of the subject.

In one embodiment of the invention, an interfering RNA is designed to target an mRNA corresponding to SEQ ID NO:1 comprising nucleotide 401, 635, 1062, 1548, 1604, 1643, 1766, 1922, 2012, 2069, 2210, 2416, 2423, 2654, 2909, 3339, 3416, 3464, 3476, 3505, 3512, 3781, 3782, 3881, 4064, 4158, 4411, 4487, 4904, 4905, 4909, 3329, 2323 or 2887.

The present invention further provides for administering a second interfering RNA to a subject in addition to a first interfering RNA. The method comprises administering to the subject a second interfering RNA having a length of 19 to 49 nucleotides and comprising a sense nucleotide strand, an antisense nucleotide strand, and a region of at least near-perfect complementarity of at least 19 nucleotides; wherein the antisense strand of the second interfering RNA hybridizes under physiological conditions to a second portion of mRNA corresponding to SEQ ID NO:1 and the antisense strand has a region of at least near-perfect contiguous complementarity of at least 19 nucleotides with the second hybridizing portion of mRNA corresponding to SEQ ID NO:1. Further, a third, fourth, or fifth, etc. interfering RNA may be administered in a similar manner.

Another embodiment of the invention is a method of attenuating expression of IGFIR mRNA in a subject comprising administering to the subject a composition comprising an effective amount of single-stranded interfering RNA having a length of 19 to 49 nucleotides and a pharmaceutically acceptable carrier.

For attenuating expression of IGF1R mRNA, the single-stranded interfering RNA hybridizes under physiological conditions to a portion of mRNA corresponding to SEQ ID NO:1 comprising nucleotide 401, 635, 1062, 1548, 1604, 1643, 1766, 1922, 2012, 2069, 2210, 2416, 2423, 2654, 2909, 3339, 3416, 3464, 3476, 3505, 3512, 3781, 3782, 3881, 4064, 4158, 4411, 4487, 4904, 4905, 4909, 3329, 2323 or 2887, and the interfering RNA has a region of at least near-perfect contiguous complementarity of at least 19 nucleotides with the hybridizing portion of mRNA corresponding to SEQ ID NO:1. Expression of IGF1R mRNA is thereby attenuated.

A further embodiment of the invention is a method of treating ocular angiogenesis in a subject in need thereof. The method comprises administering to an eye of the subject a composition comprising an effective amount of interfering RNA having a length of 19 to 49 nucleotides and a pharmaceutically acceptable carrier, the interfering RNA comprising a sense nucleotide strand, an antisense nucleotide strand, and a region of at least near-perfect contiguous complementarity of at least 19 nucleotides. The antisense strand hybridizes under physiological conditions to a portion of mRNA corresponding to SEQ ID NO:1 and has a region of at least near-perfect contiguous complementarity of at least 19 nucleotides with the hybridizing portion of mRNA corresponding to SEQ ID NO:1. The ocular angiogenesis is treated thereby.

Another embodiment of the invention is a method of treating ocular angiogenesis in a subject in need thereof, the method comprising administering to an eye of the subject a composition comprising an effective amount of interfering RNA having a length of 19 to 49 nucleotides and a pharmaceutically acceptable carrier, the interfering RNA comprising a region of at least 13 contiguous nucleotides having at least 90% sequence complementarity to, or at least 90% sequence identity with, the penultimate 13 nucleotides of the 3' end of an mRNA corresponding to any one of SEQ ID NO:2 and SEQ ID NO:8 - SEQ ID NO:40, wherein the ocular angiogenesis is treated thereby.

Another embodiment of the invention is a method of attenuating expression of an IGF1R mRNA in a subject, comprising administering to the subject a composition comprising an effective amount of interfering RNA having a length of 19 to 49 nucleotides and a pharmaceutically acceptable carrier, where the interfering RNA comprises a region of at least 13 contiguous nucleotides having at least 90% sequence complementarity to, or at least 90% sequence identity with, the penultimate 13 nucleotides of the 3' end of an mRNA corresponding to any one of SEQ ID NO:2 and SEQ ID NO:8 - SEQ ID NO:40.

In a further embodiment of the present invention, the region of contiguous nucleotides is a region of at least 14 contiguous nucleotides having at least 85% sequence complementarity to, or at least 85% sequence identity with, the penultimate 14 nucleotides of the 3' end of an mRNA corresponding to the sequence of the sequence identifier. In yet another embodiment of the invention, the region of contiguous nucleotides is a region of at least 15, 16, 17, or 18 contiguous nucleotides having at least 80% sequence complementarity to, or at least 80% sequence identity with, the penultimate 15, 16, 17, or 18 nucleotides, respectively, of the 3' end of an mRNA corresponding to the sequence identified by the sequence identifier.

A further embodiment of the invention is a method of treating ocular angiogenesis in a subject in need thereof, the method comprising administering to the subject a composition comprising a double stranded siRNA molecule that down regulates expression of an IGF1R gene via RNA interference, wherein each strand of the siRNA molecule is independently about 19 to about 27 nucleotides in length; and one strand of the siRNA molecule comprises a nucleotide sequence having substantial complementarity to an mRNA corresponding to the IGF1R gene, respectively, so that the siRNA molecule directs cleavage of the mRNA via RNA interference.

A composition comprising interfering RNA having a length of 19 to 49 nucleotides and having a nucleotide sequence of any one of SEQ ID NO:2, and SEQ ID NO:8 - SEQ ID NO:40, or a complement thereof, and a pharmaceutically acceptable carrier is an embodiment of the present invention. In one embodiment, the interfering RNA is isolated. The term "isolated" means that the interfering RNA is free of its total natural mileau.

Another embodiment of the invention is a composition comprising a double stranded siRNA molecule that down regulates expression of an IGF1R gene via RNA interference, wherein each strand of the siRNA molecule is independently about 19 to about 27 nucleotides in length; and one strand of the siRNA molecule comprises a nucleotide sequence has substantial complementarity to an mRNA corresponding to the IGF1R gene, respectively, so that the siRNA molecule directs cleavage of the mRNA via RNA interference.

The present invention provides an advantage over small molecule inhibitors ofIGF-IR since an undesirable side effect of current small molecule therapies, e.g., lack of specificity, can be overcome.

Use of any of the embodiments as described herein in the preparation of a medicament for attenuating expression of IGF1R mRNA is also an embodiment of the present invention.

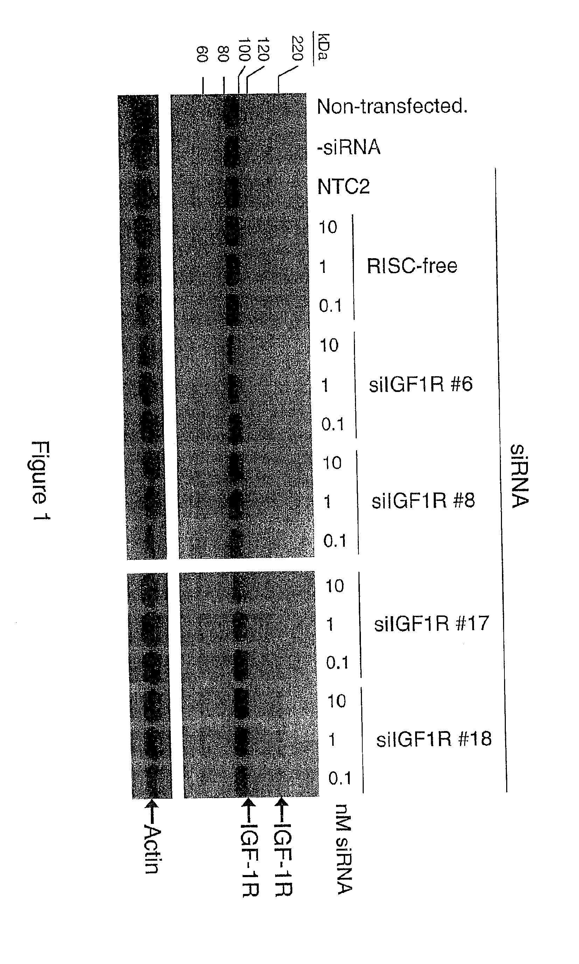

### BRIEF DESCRIPTION OF THE DRAWING

In order that the manner in which the above recited and other enhancements and objects of the invention are obtained, a more particular description of the invention briefly described above will be rendered by reference to specific embodiments thereof, which are illustrated, in the appended drawings. Understanding that these drawings depict only typical embodiments of the invention and are therefore not to be considered limiting of its scope, the invention will be described with additional specificity and detail through the use of the accompanying drawings in which:

The figure provides an IGF-1Rβ western blot of HeLa cells transfected with IGF1R siRNAs #6, #8, #17, and #18, and a RISC-free control siRNA, each at 10 nM, 1 nM, and 0.1 nM; a non-targeting control siRNA (NTC2) at 10 nM; and a buffer control (-siRNA). The arrows indicate the positions of the 97-kDa IGF-1Rβ, 200-kDa IGF-1R precursor, and 42-kDa actin bands.

### DETAILED DESCRIPTION OF THE INVENTION

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

The following definitions and explanations are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition.

As used herein, all percentages are percentages by weight, unless stated otherwise.

As used herein, a "fluid" is a continuous, amorphous substance whose molecules move freely past one another and that has the tendency to assume the shape of its container, for example, a liquid or a gas.

As used herein, the term "health care provider" is known in the art and specifically includes a physician, a person with authority to prescribe a medication (whether directly or indirectly), and a veterinarian. In certain embodiments, a health care provider includes an individual that provides a medication without prescription, such as in providing an over-the-counter medication.

As used herein, the terms "identifying subjects" and "diagnosing" are used interchangeably with regard to the detection of a "predisposition", "increased propensity", "risk", "increased risk", and the like.

As used herein, the term "other retinal or optic nerve disease" means and refers to at least one of age-related macular degeneration, cataract, acute ischemic optic neuropathy (AION), commotio retinae, retinal detachment, retinal tears or holes, diabetic retinopathy and iatrogenic retinopathy and other ischemic retinopathies or optic neuropathies, myopia, retinitis pigmentosa, and/or the like.

RNA interference (RNAi) is a process by which double-stranded RNA (dsRNA) is used to silence gene expression. While not wanting to be bound by theory, RNAi begins with the cleavage of longer dsRNAs into small interfering RNAs (siRNAs) by an RNaseIII-like enzyme, dicer. SiRNAs are dsRNAs that are usually about 19 to 28 nucleotides, or 20 to 25 nucleotides, or 21 to 22 nucleotides in length and often contain 2-nucleotide 3' overhangs, and 5' phosphate and 3' hydroxyl termini. One strand of the siRNA is incorporated into a ribonucleoprotein complex known as the RNA-induced silencing complex (RISC). RISC uses this siRNA strand to identify mRNA molecules that are at least partially complementary to the incorporated siRNA strand, and then cleaves these target mRNAs or inhibits their translation. Therefore, the siRNA strand that is incorporated into RISC is known as the guide strand or the antisense strand. The other siRNA strand, known as the passenger strand or the sense strand, is eliminated from the siRNA and is at least partially homologous to the target mRNA. Those of skill in the art will recognize that, in principle, either strand of an siRNA can be incorporated into RISC and function as a guide strand. However, siRNA design (e.g., decreased siRNA duplex stability at the 5' end of the antisense strand) can favor incorporation of the antisense strand into RISC.

RISC-mediated cleavage of mRNAs having a sequence at least partially complementary to the guide strand leads to a decrease in the steady state level of that mRNA and of the corresponding protein encoded by this mRNA. Alternatively, RISC can also decrease expression of the corresponding protein via translational repression without cleavage of the target mRNA. Other RNA molecules and RNA-like molecules can also interact with RISC and silence gene expression. Examples of other RNA molecules that can interact with RISC include short hairpin RNAs (shRNAs), single-stranded siRNAs, microRNAs (miRNAs), and dicer-substrate 27-mer duplexes. The term "siRNA" as used herein refers to a double-stranded interfering RNA unless otherwise noted. Examples of RNA-like molecules that can interact with RISC include RNA molecules containing one or more chemically modified nucleotides, one or more deoxyribonucleotides, and/or one or more non-phosphodiester linkages. For purposes of the present discussion, all RNA or RNA-like molecules that can interact with RISC and participate in RISC-mediated changes in gene expression will be referred to as "interfering RNAs." SiRNAs, shRNAs, miRNAs, and dicer-substrate 27-mer duplexes are, therefore, subsets of "interfering RNAs."

Interfering RNA of embodiments of the invention appear to act in a catalytic manner for cleavage of target mRNA, i.e., interfering RNA is able to effect inhibition of target mRNA in substoichiometric amounts. As compared to antisense therapies, significantly less interfering RNA is required to provide a therapeutic effect under such cleavage conditions.

The present invention relates to the use of interfering RNA to inhibit the expression of insulin-like growth factor-1 receptor (IGF-1R) mRNA, thus interfering with ligand binding and interfering with subsequent proliferation and angiogenesis. According to the present invention, interfering RNAs provided exogenously or expressed endogenously effect silencing of IGF1R expression in ocular tissues.

Nucleic acid sequences cited herein are written in a 5' to 3' direction unless indicated otherwise. The term "nucleic acid," as used herein, refers to either DNA or RNA or a modified form thereof comprising the purine or pyrimidine bases present in DNA (adenine "A," cytosine "C," guanine "G," thymine "T") or in RNA (adenine "A," cytosine "C," guanine "G," uracil "U"). Interfering RNAs provided herein may comprise "T" bases, particularly at 3' ends, even though "T" bases do not naturally occur in RNA. "Nucleic acid" includes the terms "oligonucleotide" and "polynucleotide" and can refer to a single-stranded molecule or a double-stranded molecule. A double-stranded molecule is formed by Watson-Crick base pairing between A and T bases, C and G bases, and between A and U bases. The strands of a double-stranded molecule may have partial, substantial or full complementarity to each other and will form a duplex hybrid, the strength of bonding of which is dependent upon the nature and degree of complementarity of the sequence of bases.

An mRNA sequence is readily deduced from the sequence of the corresponding DNA sequence. For example, SEQ ID NO:1 provides the sense strand sequence of DNA corresponding to the mRNA for IGF1R. The mRNA sequence is identical to the DNA sense strand sequence with the "T" bases replaced with "U" bases. Therefore, the mRNA sequence of IGF1R is known from SEQ ID NO:1.

*Insulin-like Growth Factor-1 Receptor mRNA (IGF1R):* IGF-1R is a member of the receptor tyrosine kinase family. Proteolytic cleavage of the IGF-1R precursor generates the extracellular, ligand-binding α subunit and the transmembrane β subunit, which contains the intracellular tyrosine kinase domain. IGF-1R comprises two α and two β subunits linked by disulfide bonds. Ligand binding triggers auto-transphosphorylation promoting proliferation and cell survival.

The biological activities of insulin growth factor-1 are mediated via IGF-1R. An increase in IGF-1 has been observed in ocular fluids and tissues from patients with advancing diabetic retinopathy, Various proangiogenic growth factors including insulin-like growth factor-1 have been found in tissues and fluids from patients with ocular angiogenesis. Patients treated subcutaneously with octreotide, a somatostatin analog that inhibits the GH/IGF1 axis, show empirical improvement in DME and PDR. In the mouse OIR model, treatment with a GH inhibitor or an IGF-1R antagonist significantly decreases retinal neovascularization. IGF-1 stimulates retinal endothelial production of vascular endothelial growth factor *in vitro.* In a mouse diabetic model, plasmid-mediated IGF-1 therapy reversed diabetic-increased angiogenesis and arterial flow. Therefore, inhibition of IGF-1R expression is provided herein for treating ocular angiogenesis including pre-angiogenic and angiogenic cellular activity.

The GenBank database of the National Center for Biotechnology Information at nebi.nlm.nih.gov provides the DNA sequence for IGF1R as accession no. NM_000875, provided in the "Sequence Listing" as SEQ ID NO:1. SEQ ID NO:1 provides the sense strand sequence of DNA that corresponds to the mRNA encoding IGF1R (with the exception of "T" bases for "U" bases). The coding sequence for IGF1R is from nucleotides 46-4149.

Equivalents of the above-cited IGF1R mRNA sequence are alternative splice forms, allelic forms, isozymes, or a cognate thereof. A cognate is an IGFIR mRNA from another mammalian species that is homologous to SEQ ID NO:1 (an ortholog).

*Attenuating expression of an mRNA:* The phrase, "attenuating expression of an mRNA," as used herein, means administering or expressing an amount of interfering RNA (e.g., an siRNA) to reduce translation of the target mRNA into protein, either through mRNA cleavage or through direct inhibition of translation. The reduction in expression of the target mRNA or the corresponding protein is commonly referred to as "knock-down" and is reported relative to levels present following administration or expression of a non-targeting control RNA (e.g., a non-targeting control siRNA). Knock-down of expression of an amount including and between 50% and 100% is contemplated by embodiments herein. However, it is not necessary that such knock-down levels be achieved for purposes of the present invention. In one embodiment, a single interfering RNA targeting IGF1R is administered to decrease production of IGF1R, thereby inhibiting the IGF1R signaling pathway. In other embodiments, two or more interfering RNAs targeting the IGF1R mRNA are administered to decrease expression. In still other embodiments, a first interfering RNA targeting the IGFIR mRNA and a second interfering RNA targeting another receptor tyrosine kinase mRNA are administered to effect a lowering of ocular pre-angiogenic and angiogenic cellular activity.

Knock-down is commonly assessed by measuring the mRNA levels using quantitative polymerase chain reaction (qPCR) amplification or by measuring protein levels by western blot or enzyme-linked immunosorbent assay (ELISA). Analyzing the protein level provides an assessment of both mRNA cleavage as well as translation inhibition. Further techniques for measuring knock-down include RNA solution hybridization, nuclease protection, northern hybridization, gene expression monitoring with a microarray, antibody binding, radioimmunoassay, and fluorescence activated cell analysis.

Inhibition of targets cited herein is also inferred in a human or mammal by observing an improvement in an ocular angiogenesis symptom such as improvement in retinal edema, diabetic retinopathy, retinal ischemia, or in posterior segment neovascularization (PSNV), for example.

*Interfering RNA:* In one embodiment of the invention, interfering RNA (e.g., siRNA) has a sense strand and an antisense strand, and the sense and antisense strands comprise a region of at least near-perfect contiguous complementarity of at least 19 nucleotides. In a further embodiment of the invention, interfering RNA (e.g., siRNA) has a sense strand and an antisense strand, and the antisense strand comprises a region of at least near-perfect contiguous complementarity of at least 19 nucleotides to a target sequence of IGF1R mRNA, and the sense strand comprises a region of at least near-perfect contiguous identity of at least 19 nucleotides with a target sequence of IGF1R mRNA, respectively. In a further embodiment of the invention, the interfering RNA comprises a region of at least 13, 14, 15, 16, 17, or 18 contiguous nucleotides having percentages of sequence complementarity to or, having percentages of sequence identity with, the penultimate 13, 14, 15, 16, 17, or 18 nucleotides, respectively, of the 3' end of an mRNA corresponding to the corresponding target sequence within an mRNA.

The length of each strand of the interfering RNA comprises 19 to 49 nucleotides, and may comprise a length of 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or 49 nucleotides.

The antisense strand of an siRNA is the active guiding agent of the siRNA in that the antisense strand is incorporated into RISC, thus allowing RISC to identify target mRNAs with at least partial complementary to the antisense siRNA strand for cleavage or translational repression.

In embodiments of the present invention, interfering RNA target sequences (e.g., siRNA target sequences) within a target mRNA sequence are selected using available design tools. Interfering RNAs corresponding to a IGF1R target sequence are then tested by transfection of cells expressing the target mRNA followed by assessment of knockdown as described above.

Techniques for selecting target sequences for siRNAs are provided by Tuschl, T. et al., "The siRNA User Guide," revised May 6, 2004, available on the Rockefeller University web site; by Technical Bulletin #506, "siRNA Design Guidelines," Ambion Inc. at Ambion's web site; and by other web-based design tools at, for example, the Invitrogen, Dharmacon, Integrated DNA Technologies, Genscript, or Proligo web sites. Initial search parameters can include G/C contents between 35% and 55% and siRNA lengths between 19 and 27 nucleotides. The target sequence may be located in the coding region or in the 5' or 3' untranslated regions of the mRNA.

An embodiment of a 19-nucleotide DNA target sequence for IGF1R mRNA is present at nucleotides 401 to 419 of SEQ ID NO:1:

| | |
|---|---|
| **5'- TCTTCGAGATGACCAATCT - 3'** | **SEQ ID NO:2.** |

An siRNA of the invention for targeting a corresponding mRNA sequence of SEQ ID NO:2 and having 21-nucleotide strands and a 2-nucleotide 3' overhang is:

| | |
|---|---|
| **5'- UCUUCGAGAUGACCAAUCUNN-3'** | **SEQ ID NO:3** |
| **3' -NNAGAAGCUCUACUGGUUAGA-5'** | **SEQ ID NO:4.** |

Each "N" residue can be any nucleotide (A, C, G, U, T) or modified nucleotide. The 3' end can have a number of "N" residues between and including 1, 2, 3, 4, 5, and 6. The "N" residues on either strand can be the same residue (e.g., UU, AA, CC, GG, or TT) or they can be different (e.g., AC, AG, AU, CA, CG, CU, GA, GC, GU, UA, UC, or UG). The 3' overhangs can be the same or they can be different. In one embodiment, both strands have a 3'UU overhang.

An siRNA of the invention for targeting a corresponding mRNA sequence of SEQ ID NO:2 and having 21-nucleotide strands and a 3'UU overhang on each strand is:

| | |
|---|---|
| **5'-UCUUCGAGAUGACCAAUCUUU-3'** | **SEQ ID NO:5** |
| **3' -UUAGAAGCUCUACUGGUUAGA-5'** | **SEQ ID NO:6.** |

The interfering RNA may also have a 5' overhang of nucleotides or it may have blunt ends, An siRNA of the invention for targeting a corresponding mRNA sequence of SEQ ID NO:2 and having 19-nucleotide strands and blunt ends is:

| | |
|---|---|
| **5'- UCUUCGAGAUGACCAAUCU -3'** | **SEQ ID NO:41** |
| **3'- AGAAGCUCUACUGGUUAGA -5'** | **SEQ ID NO:42.** |

The strands of a double-stranded interfering RNA (e.g., an siRNA) may be connected to form a hairpin or stem-loop structure (e.g., an shRNA). An shRNA of the invention targeting a corresponding mRNA sequence of SEQ ID NO: and having a 19 bp double-stranded stem region and a 3'UU overhang is: 
NNN
/ \
5'-UCUUCGAGAUGACCAAUCU N
3'-UUAGAAGCUCUACUGGUUAGA N SEQ ID NO:7.
\ /
NNN
 N is a nucleotide A, T, C, G, U, or a modified form known by one of ordinary skill in the art. The number of nucleotides N in the loop is a number between and including 3 to 23, or 5 to 15, or 7 to 13, or 4 to 9, or 9 to 11, or the number of nucleotides N is 9. Some of the nucleotides in the loop can be involved in base-pair interactions with other nucleotides in the loop. Examples of oligonucleotide sequences that can be used to form the loop include 5'-UUCAAGAGA-3' (Brummelkamp, T.R. et al. (2002) Science 296: 550) and 5'-UUUGUGUAG-3' (Castanotto, D. et al. (2002) RNA 8:1454). It will be recognized by one of skill in the art that the resulting single chain oligonucleotide forms a stem-loop or hairpin structure comprising a double-stranded region capable of interacting with the RNAi machinery.

The siRNA target sequence identified above can be extended at the 3' end to facilitate the design of dicer-substrate 27-mer duplexes. Extension of the 19-nucleotide DNA target sequence (SEQ ID NO:2) identified in the IGF1R DNA sequence (SEQ ID NO:1) by 6 nucleotides yields a 25-nucleotide DNA target sequence present at nucleotides 401 to 425 of SEQ ID NO:1:

| | |
|---|---|
| **5'- TCTTCGAGATGACCAATCTCAAGGA -3'** | **SEQ ID NO:43.** |

A dicer-substrate 27-mer duplex of the invention for targeting a corresponding mRNA sequence of SEQ ID NO:43 is:

| | |
|---|---|
| **5'- UCUUCGAGAUGACCAAUCUCAAGGA -3'** | **SEQ ID NO : 44** |
| **3'- UUAGAAGCUCUACUGGUUAGAGUUCCU -5'** | **SEQ ID NO:45.** |

The two nucleotides at the 3' end of the sense strand (i.e., the GA nucleotides of SEQ ID NO:44) may be deoxynucleotides for enhanced processing. Design of dicer-substrate 27-mer duplexes from 19-21 nucleotide target sequences, such as provided herein, is further discussed by the Integrated DNA Technologies (IDT) website and by Kim, D.-H. et al., (February, 2005) Nature Biotechnology 23:2; 222-226.

When interfering RNAs are produced by chemical synthesis, phosphorylation at the 5' position of the nucleotide at the 5' end of one or both strands (when present) can enhance siRNA efficacy and specificity of the bound RISC complex but is not required since phosphorylation can occur intracellularly.

Table 1 lists examples of IGF1R DNA target sequences of SEQ ID NO:1 from which siRNAs of the present invention are designed in a manner as set forth above. IGF1R encodes insulin-like growth factor-1 receptor, as noted above.

**Table 1. IGFIR Target Sequences for siRNAs**

| **IGF1R Target Sequence** | **# of Starting Nucleotide with reference to SEQ ID NO : 1** | **SEQ ID NO:** |
|---|---|---|
| **TCTTCGAGATGACCAATCT** | **401** | **2** |
| **TCAACAATGAGTACAACTA** | **635** | **8** |
| **GACCATTGATTCTGTTACT** | **1062** | **9** |
| **GAAGAATCGCATCATCATA** | **1548** | **10** |
| **TCATCAGCTTCACCGTTTA** | **1604** | **11** |
| **AGAATGTCACAGAGTATGA** | **1643** | **12** |
| **GGACTCAGTACGCCGTTTA** | **1766** | **13** |
| **AGTTAATCGTGAAGTGGAA** | **1922** | **14** |
| **ACCTTTACCGGCACAATTA** | **2012** | **15** |
| **ACGGCACCATCGACATTGA** | **2069** | **16** |
| **TTGAGAATTTCCTGCACAA** | **2210** | **17** |
| **TCTAACCTTCGGCCTTTCA** | **2416** | **18** |
| **TTCGGCCTTTCACATTGTA** | **2423** | **19** |
| **GATCACAAGTTGAGGATCA** | **2654** | **20** |
| **TGTACGTCTTCCATAGAAA** | **2909** | **21** |
| **GGAGAATAATCCAGTCCTA** | **3339** | **22** |
| **CATACCTCAACGCCAA** | **3416** | **23** |
| **ATTGCATGGTAGCCGAAGA** | **3464** | **24** |
| **CCGAAGATTTCACAGTCAA** | **3476** | **25** |
| **TTTGGTATGACGCGAGATA** | **3505** | **26** |
| **TGACGCGAGATATCTATGA** | **3512** | **27** |
| **CGCATGTGCTGGCAGTATA** | **3781** | **28** |
| **GCATGTGCTGGCAGTATAA** | **3782** | **29** |
| **TCTACTACAGCGAGGAGAA** | **3881** | **30** |
| **TCGACGAGAGACAGCCTTA** | **4064** | **31** |
| **TCCTGAATCTGTGCAAACA** | **4158** | **32** |
| **TAATAGCAACAGAGCACTT** | **4411** | **33** |
| **CTCTGCTTCATAACGGAAA** | **4487** | **34** |
| **TCATTGCTTCTGACTAGAT** | **4904** | **35** |
| **CATTGCTTCTGACTAGATT** | **4905** | **36** |
| **GCTTCTGACTAGATTATTA** | **4909** | **37** |
| **GGCCAGAAATGGAGAATAA** | **3329** | **38** |
| **GCAGACACCTACAACATCA** | **2323** | **39** |
| **GTGGGAGGGTTGGTGATTA** | **2887** | **40** |
| | | |

As cited in the examples above, one of skill in the art is able to use the target sequence information provided in Table 1 to design interfering RNAs having a length shorter or longer than the sequences provided in the table and by referring to the sequence position in SEQ ID NO:1 and adding or deleting nucleotides complementary or near complementary to SEQ ID NO:1.

The target RNA cleavage reaction guided by siRNAs and other forms of interfering RNA is highly sequence specific. In general, siRNA containing a sense nucleotide strand identical in sequence to a portion of the target mRNA and an antisense nucleotide strand exactly complementary to a portion of the target mRNA are siRNA embodiments for inhibition of mRNAs cited herein. However, 100% sequence complementarity between the antisense siRNA strand and the target mRNA, or between the antisense siRNA strand and the sense siRNA strand, is not required to practice the present invention. Thus, for example, the invention allows for sequence variations that might be expected due to genetic mutation, strain polymorphism, or evolutionary divergence.

In one embodiment of the invention, the antisense strand of the siRNA has at least near-perfect contiguous complementarity of at least 19 nucleotides with the target mRNA. "Near-perfect," as used herein, means the antisense strand of the siRNA is "substantially complementary to," and the sense strand of the siRNA is "substantially identical to" at least a portion of the target mRNA. "Identity," as known by one of ordinary skill in the art, is the degree of sequence relatedness between nucleotide sequences as determined by matching the order and identity of nucleotides between the sequences. In one embodiment, the antisense strand of an siRNA having 80% and between 80% up to 100% complementarity, for example, 85%, 90% or 95% complementarity, to the target mRNA sequence are considered near-perfect complementarity and may be used in the present invention. "Perfect" contiguous complementarity is standard Watson-Crick base pairing of adjacent base pairs. "At least near-perfect" contiguous complementarity includes "perfect" complementarity as used herein. Computer methods for determining identity or complementarity are designed to identify the greatest degree of matching of nucleotide sequences, for example, BLASTN (Altschul, S.F., et al. (1990) J. Mol. Biol. 215:403-410).

The term "percent identity" describes the percentage of contiguous nucleotides in a first nucleic acid molecule that is the same as in a set of contiguous nucleotides of the same length in a second nucleic acid molecule. The term "percent complementarity" describes the percentage of contiguous nucleotides in a first nucleic acid molecule that can base pair in the Watson-Crick sense with a set of contiguous nucleotides in a second nucleic acid molecule.

The relationship between a target mRNA (sense strand) and one strand of an siRNA (the sense strand) is that of identity. The sense strand of an siRNA is also called a passenger strand, if present. The relationship between a target mRNA (sense strand) and the other strand of an siRNA (the antisense strand) is that of complementarity. The antisense strand of an siRNA is also called a guide strand.

The penultimate base in a nucleic acid sequence that is written in a 5' to 3' direction is the next to the last base, i.e., the base next to the 3' base. The penultimate 13 bases of a nucleic acid sequence written in a 5' to 3' direction are the last 13 bases of a sequence next to the 3' base and not including the 3' base. Similarly, the penultimate 14, 15, 16, 17, or 18 bases of a nucleic acid sequence written in a 5' to 3' direction are the last 14, 15, 16, 17, or 18 bases of a sequence, respectively, next to the 3' base and not including the 3' base.

The phrase "a region of at least 13 contiguous nucleotides having at least 90% sequence complementarity to, or at least 90% sequence identity with, the penultimate 13 nucleotides of the 3' end of an mRNA corresponding to any one of (a sequence identifier)" allows a one nucleotide substitution. Two nucleotide substitutions (i.e., 11/13 = 85% identity/complementarity) are not included in such a phrase.

In one embodiment of the invention, the region of contiguous nucleotides is a region of at least 14 contiguous nucleotides having at least 85% sequence complementarity to, or at least 85% sequence identity with, the penultimate 14 nucleotides of the 3' end of an mRNA corresponding to the sequence identified by each sequence identifier. Two nucleotide substitutions (i.e., 12/14 = 86% identity/complementarity) are included in such a phrase.

In a further embodiment of the invention, the region of contiguous nucleotides is a region of at least 15, 16, 17, or 18 contiguous nucleotides having at least 80% sequence complementarity to, or at least 80% sequence identity with, the penultimate 14 nucleotides of the 3' end of an mRNA corresponding to the sequence of the sequence identifier. Three nucleotide substitutions are included in such a phrase.

The target sequence in the mRNAs corresponding to SEQ ID NO:1 may be in the 5' or 3' untranslated regions of the mRNA as well as in the coding region of the mRNA.

One or both of the strands of double-stranded interfering RNA may have a 3' overhang of from 1 to 6 nucleotides, which may be ribonucleotides or deoxyribonucleotides or a mixture thereof. The nucleotides of the overhang are not base-paired. In one embodiment of the invention, the interfering RNA comprises a 3' overhang of TT or UU. In another embodiment of the invention, the interfering RNA comprises at least one blunt end. The termini usually have a 5' phosphate group or a 3' hydroxyl group. In other embodiments, the antisense strand has a 5' phosphate group, and the sense strand has a 5' hydroxyl group. In still other embodiments, the termini are further modified by covalent addition of other molecules or functional groups.

The sense and antisense strands of the double-stranded siRNA may be in a duplex formation of two single strands as described above or may be a single molecule where the regions of complementarity are base-paired and are covalently linked by a hairpin loop so as to form a single strand. It is believed that the hairpin is cleaved intracellularly by a protein termed dicer to form an interfering RNA of two individual base-paired RNA molecules.

Interfering RNAs may differ from naturally-occurring RNA by the addition, deletion, substitution or modification of one or more nucleotides. Non-nucleotide material may be bound to the interfering RNA, either at the 5' end, the 3' end, or internally. Such modifications are commonly designed to increase the nuclease resistance of the interfering RNAs, to improve cellular uptake, to enhance cellular targeting, to assist in tracing the interfering RNA, to further improve stability, or to reduce the potential for activation of the interferon pathway. For example, interfering RNAs may comprise a purine nucleotide at the ends of overhangs. Conjugation of cholesterol to the 3' end of the sense strand of an siRNA molecule by means of a pyrrolidine linker, for example, also provides stability to an siRNA.

Further modifications include a 3' terminal biotin molecule, a peptide known to have cell-penetrating properties, a nanoparticle, a peptidomimetic, a fluorescent dye, or a dendrimer, for example.

Nucleotides may be modified on their base portion, on their sugar portion, or on the phosphate portion of the molecule and function in embodiments of the present invention. Modifications include substitutions with alkyl, alkoxy, amino, deaza, halo, hydroxyl, thiol groups, or a combination thereof, for example. Nucleotides may be substituted with analogs with greater stability such as replacing a ribonucleotide with a deoxyribonucleotide, or having sugar modifications such as 2' OH groups replaced by 2' amino groups, 2' O-methyl groups, 2' methoxyethyl groups, or a 2'-O, 4'-C methylene bridge, for example. Examples of a purine or pyrimidine analog of nucleotides include a xanthine, a hypoxanthine, an azapurine, a methylthioadenine, 7-deaza-adenosine and O- and N-modified nucleotides. The phosphate group of the nucleotide may be modified by substituting one or more of the oxygens of the phosphate group with nitrogen or with sulfur (phosphorothioates). Modifications are useful, for example, to enhance function, to improve stability or permeability, or to direct localization or targeting.

There may be a region or regions of the antisense interfering RNA strand that is (are) not complementary to a portion of SEQ ID NO:1. Non-complementary regions may be at the 3', 5' or both ends of a complementary region or between two complementary regions.

Interfering RNAs may be generated exogenously by chemical synthesis, by *in vitro* transcription, or by cleavage of longer double-stranded RNA with dicer or another appropriate nuclease with similar activity. Chemically synthesized interfering RNAs, produced from protected ribonucleoside phosphoramidites using a conventional DNA/RNA synthesizer, may be obtained from commercial suppliers such as Ambion Inc. (Austin, TX), Invitrogen (Carlsbad, CA), or Dharmacon (Lafayette, CO). Interfering RNAs are purified by extraction with a solvent or resin, precipitation, electrophoresis, chromatography, or a combination thereof, for example. Alternatively, interfering RNA may be used with little if any purification to avoid losses due to sample processing.

Interfering RNAs can also be expressed endogenously from plasmid or viral expression vectors or from minimal expression cassettes, for example, PCR generated fragments comprising one or more promoters and an appropriate template or templates for the interfering RNA. Examples of commercially available plasmid-based expression vectors for shRNA include members of the pSilencer series (Ambion, Austin, TX) and pCpG-siRNA (InvivoGen, San Diego, CA). Viral vectors for expression of interfering RNA may be derived from a variety of viruses including adenovirus, adeno-associated virus, lentivirus (e.g., HIV, FIV, and EIAV), and herpes virus. Examples of commercially available viral vectors for shRNA expression include pSilencer adeno (Ambion, Austin, TX) and pLenti6/BLOCK-iT™-DEST (Invitrogen, Carlsbad, CA). Selection of viral vectors, methods for expressing the interfering RNA from the vector and methods of delivering the viral vector are within the ordinary skill of one in the art. Examples of kits for production of PCR-generated shRNA expression cassettes include Silencer Express (Ambion, Austin, TX) and siXpress (Mirus, Madison, WI). A first interfering RNA may be administered via *in vivo* expression from a first expression vector capable of expressing the first interfering RNA and a second interfering RNA may be administered via *in vivo* expression from a second expression vector capable of expressing the second interfering RNA, or both interfering RNAs may be administered via *in vivo* expression from a single expression vector capable of expressing both interfering RNAs.

Interfering RNAs may be expressed from a variety of eukaryotic promoters known to those of ordinary skill in the art, including pol III promoters, such as the U6 or H1 promoters, or pol II promoters, such as the cytomegalovirus promoter. Those of skill in the art will recognize that these promoters can also be adapted to allow inducible expression of the interfering RNA.

*Hybridization under Physiological Conditions:* In certain embodiments of the present invention, an antisense strand of an interfering RNA hybridizes with an mRNA *in vivo* as part of the RISC complex.

"Hybridization" refers to a process in which single-stranded nucleic acids with complementary or near-complementary base sequences interact to form hydrogen-bonded complexes called hybrids. Hybridization reactions are sensitive and selective. *In vitro,* the specificity of hybridization (i.e., stringency) is controlled by the concentrations of salt or formamide in prehybridization and hybridization solutions, for example, and by the hybridization temperature; such procedures are well known in the art. In particular, stringency is increased by reducing the concentration of salt, increasing the concentration of formamide, or raising the hybridization temperature.

For example, high stringency conditions could occur at about 50% formamide at 37 °C to 42 °C. Reduced stringency conditions could occur at about 35% to 25% formamide at 30 °C to 35 °C. Examples of stringency conditions for hybridization are provided in Sambrook, J., 1989, Molecular Cloning: A Laboratory Manual, Cold Spring Harbor Laboratory Press, Cold Spring Harbor, N.Y. Further examples of stringent hybridization conditions include 400 mM NaCl, 40 mM PIPES pH 6.4, 1 mM EDTA, 50 °C or 70 °C for 12-16 hours followed by washing, or hybridization at 70 °C in 1XSSC or 50 °C in 1XSSC, 50% formamide followed by washing at 70 °C in 0.3XSSC, or hybridization at 70 °C in 4XSSC or 50 °C in 4XSSC, 50% formamide followed by washing at 67 °C in 1XSSC. The temperature for hybridization is about 5-10 °C less than the melting temperature (Tₘ) of the hybrid where Tₘ is determined for hybrids between 19 and 49 base pairs in length using the following calculation: Tₘ °C = 81.5 + 16.6(log₁₀[Na+]) + 0.41 (% G+C) - (600/N) where N is the number of bases in the hybrid, and [Na+] is the concentration of sodium ions in the hybridization buffer.

The above-described *in vitro* hybridization assay provides a method of predicting whether binding between a candidate siRNA and a target will have specificity. However, in the context of the RISC complex, specific cleavage of a target can also occur with an antisense strand that does not demonstrate high stringency for hybridization *in vitro.*

*Single-stranded interfering RNA:* As cited above, interfering RNAs ultimately function as single strands. Single-stranded (ss) interfering RNA has been found to effect mRNA silencing, albeit less efficiently than double-stranded siRNA. Therefore, embodiments of the present invention also provide for administration of a ss interfering RNA that hybridizes under physiological conditions to a portion of SEQ ID NO;1 and has a region of at least near-perfect contiguous complementarity of at least 19 nucleotides with the hybridizing portion of SEQ ID NO:1. The ss interfering RNA has a length of 19 to 49 nucleotides as for the ds siRNA cited above. The ss interfering RNA has a 5' phosphate or is phosphorylated *in situ* or *in vivo* at the 5' position. The term "5' phosphorylated" is used to describe, for example, polynucleotides or oligonucleotides having a phosphate group attached via ester linkage to the C5 hydroxyl of the sugar (e.g., ribose, deoxyribose, or an analog of same) at the 5' end of the polynucleotide or oligonucleotide.

SS interfering RNAs are synthesized chemically or by *in vitro* transcription or expressed endogenously from vectors or expression cassettes as for ds interfering RNAs. 5' Phosphate groups may be added via a kinase, or a 5' phosphate may be the result of nuclease cleavage of an RNA. Delivery is as for ds interfering RNAs. In one embodiment, ss interfering RNAs having protected ends and nuclease resistant modifications are administered for silencing. SS interfering RNAs may be dried for storage or dissolved in an aqueous solution. The solution may contain buffers or salts to inhibit annealing or for stabilization.

*Hairpin interfering RNA:* A hairpin interfering RNA is a single molecule (e.g., a single oligonucleotide chain) that comprises both the sense and antisense strands of an interfering RNA in a stem-loop or hairpin structure (e.g., a shRNA). For example, shRNAs can be expressed from DNA vectors in which the DNA oligonucleotides encoding a sense interfering RNA strand are linked to the DNA oligonucleotides encoding the reverse complementary antisense interfering RNA strand by a short spacer. If needed for the chosen expression vector, 3' terminal T's and nucleotides forming restriction sites may be added. The resulting RNA transcript folds back onto itself to form a stem-loop structure.

*Mode of administration:* Interfering RNA may be delivered via aerosol, buccal, dermal, intradermal, inhaling, intramuscular, intranasal, intraocular, intrapulmonary, intravenous, intraperitoneal, nasal, ocular, oral, otic, parenteral, patch, subcutaneous, sublingual, topical, or transdermal administration, for example.

Interfering RNA may be delivered directly to the eye by ocular tissue injection such as periocular, conjunctival, subtenon, intracameral, intravitreal, intraocular, subretinal, subconjunctival, retrobulbar, or intracanalicular injections; by direct application to the eye using a catheter or other placement device such as a retinal pellet, intraocular insert, suppository or an implant comprising a porous, non-porous, or gelatinous material; by topical ocular drops or ointments; or by a slow release device in the cul-de-sac or implanted adjacent to the sclera (transscleral) or within the eye. Intracameral injection may be through the cornea into the anterior chamber to allow the agent to reach the trabecular meshwork. Intracanalicular injection may be into the venous collector channels draining Schlemm's canal or into Schlemm's canal.

*Subject:* A subject in need of treatment for ocular angiogenesis or at risk for developing ocular angiogenesis is a human or other mammal having ocular angiogenesis or at risk of having ocular angiogenesis associated with undesired or inappropriate expression or activity of IGF-1R as cited herein. Ocular structures associated with such disorders may include the eye, retina, choroid, lens, cornea, trabecular meshwork, iris, optic nerve, optic nerve head, sclera, anterior or posterior segments, or ciliary body, for example. A subject may also be an ocular cell, cell culture, organ or an *ex vivo* organ or tissue.

*Formulations and Dosage:* Pharmaceutical formulations comprise interfering RNAs, or salts thereof, of the invention up to 99% by weight mixed with a physiologically acceptable carrier medium such as water, buffer, saline, glycine, hyaluronic acid, mannitol, and the like.

Interfering RNAs of the present invention are administered as solutions, suspensions, or emulsions. The following are examples of possible formulations embodied by this invention.

| | |
|---|---|
| Interfering RNA | Amount in weight % up to 99; 0.1-99; 0.1 - 50; 0.5 - 10.0 |
| Hydroxypropylmethylcellulose | 0.5 |
| Sodium chloride | 0.8 |
| Benzalkonium Chloride | 0.01 |
| EDTA | 0.01 |
| NaOH/HCl | qs pH 7.4 |
| Purified water (RNase-free) | qs 100 mL |

| | Amount in weight % |
|---|---|
| Interfering RNA | up to 99; 0.1-99; 0.1 - 50; 0.5 - 10.0 |
| Phosphate Buffered Saline | 1.0 |
| Benzalkonium Chloride | 0.01 |
| Polysorbate 80 | 0.5 |
| Purified water (RNase-free) | q.s. to 100% |

| | Amount in weight % |
|---|---|
| Interfering RNA | up to 99; 0. 1-99; 0.1 - 50; 0.5 - 10.0 |
| Monobasic sodium phosphate | 0.05 |
| Dibasic sodium phosphate (anhydrous) | 0.15 |
| Sodium chloride | 0.75 |
| Disodium EDTA | 0.05 |
| Cremophor EL | 0.1 |
| Benzalkonium chloride | 0.01 |
| HCl and/or NaOH | pH 7.3-7.4 |
| Purified water (RNase-free) | q.s. to 100% |

| | Amount in weight % |
|---|---|
| Interfering RNA | up to 99; 0.1-99; 0.1 - 50; 0,5 -10.0 |
| Phosphate Buffered Saline | 1.0 |
| Hydroxypropyl-β-cyclodextrin | 4.0 |
| Purified water (RNase-free) | q.s. to 100% |

Generally, an effective amount of the interfering RNAs of embodiments of the invention results in an extracellular concentration at the surface of the target cell of from 100 pM to 1 µM, or from 1 nM to 100 nM, or from 5 nM to about 50 nM, or to about 25 nM. The dose required to achieve this local concentration will vary depending on a number of factors including the delivery method, the site of delivery, the number of cell layers between the delivery site and the target cell or tissue, whether delivery is local or systemic, etc. The concentration at the delivery site may be considerably higher than it is at the surface of the target cell or tissue. Topical compositions are delivered to the surface of the target organ one to four times per day, or on an extended delivery schedule such as daily, weekly, bi-weekly, monthly, or longer, according to the routine discretion of a skilled clinician. The pH of the formulation is about pH 4-9, or pH 4.5 to pH 7.4.

Therapeutic treatment of patients with interfering RNAs directed against IGF1R mRNA is expected to be beneficial over small molecule treatments by increasing the duration of action, thereby allowing less frequent dosing and greater patient compliance.

An effective amount of a formulation may depend on factors such as the age, race, and sex of the subject, the severity of the ocular angiogenesis, the rate of target gene transcript/protein turnover, the interfering RNA potency, and the interfering RNA stability, for example. In one embodiment, the interfering RNA is delivered topically to a target organ and reaches the IGF1R mRNA-containing tissue such as the retina or optic nerve head at a therapeutic dose thereby ameliorating an ocular angiogenesis-associated disease process.

*Acceptable carriers:* An acceptable carrier refers to those carriers that cause at most, little to no ocular irritation, provide suitable preservation if needed, and deliver one or more interfering RNAs of the present invention in a homogenous dosage. An acceptable carrier for administration of interfering RNA of embodiments of the present invention include the cationic lipid-based transfection reagents TransIT^{®}-TKO (Mirus Corporation, Madison, WI), LIFOFECTIN®, Lipofectamine, OLIGOFECTAMINE™ (Invitrogen, Carlsbad, CA), or DHARMAFECT™ (Dharmacon, Lafayette, CO); polycations such as polyethyleneimine; cationic peptides such as Tat, polyarginine, or Penetratin (Antp peptide); or liposomes. Liposomes are formed from standard vesicle-forming lipids and a sterol, such as cholesterol, and may include a targeting molecule such as a monoclonal antibody having binding affinity for endothelial cell surface antigens, for example. Further, the liposomes may be PEGylated liposomes.

The interfering RNAs may be delivered in solution, in suspension, or in bioerodible or non-bioerodible delivery devices. The interfering RNAs can be delivered alone or as components of defined, covalent conjugates. The interfering RNAs can also be complexed with cationic lipids, cationic peptides, or cationic polymers; complexed with proteins, fusion proteins, or protein domains with nucleic acid binding properties (e.g., protamine); or encapsulated in nanoparticles or liposomes. Tissue- or cell-specific delivery can be accomplished by the inclusion of an appropriate targeting moiety such as an antibody or antibody fragment.

For ophthalmic delivery, an interfering RNA may be combined with ophthalmologically acceptable preservatives, co-solvents, surfactants, viscosity enhancers, penetration enhancers, buffers, sodium chloride, or water to form an aqueous, sterile ophthalmic suspension or solution. Solution formulations may be prepared by dissolving the interfering RNA in a physiologically acceptable isotonic aqueous buffer. Further, the solution may include an acceptable surfactant to assist in dissolving the inhibitor. Viscosity building agents, such as hydroxymethyl cellulose, hydroxyethyl cellulose, methylcellulose, polyvinylpyrrolidone, or the like may be added to the compositions of the present invention to improve the retention of the compound.

In order to prepare a sterile ophthalmic ointment formulation, the interfering RNA is combined with a preservative in an appropriate vehicle, such as mineral oil, liquid lanolin, or white petrolatum. Sterile ophthalmic gel formulations may be prepared by suspending the interfering RNA in a hydrophilic base prepared from the combination of, for example, CARBOPOL^{®}-940 (BF Goodrich, Charlotte, NC), or the like, according to methods known in the art. VISCOAT^{®} (Alcon Laboratories, Inc., Fort Worth, TX) may be used for intraocular injection, for example. Other compositions of the present invention may contain penetration enhancing agents such as cremephor and TWEEN^{®} 80 (polyoxyethylene sorbitan monolaureate, Sigma Aldrich, St. Louis, MO), in the event the interfering RNA is less penetrating in the eye.

*Kits*: Embodiments of the present invention provide a kit that includes reagents for attenuating the expression of an mRNA as cited herein in a cell. The kit contains an siRNA or an shRNA expression vector. For siRNAs and non-viral shRNA expression vectors the kit also contains a transfection reagent or other suitable delivery vehicle. For viral shRNA expression vectors, the kit may contain the viral vector and/or the necessary components for viral vector production (e.g., a packaging cell line as well as a vector comprising the viral vector template and additional helper vectors for packaging). The kit may also contain positive and negative control siRNAs or shRNA expression vectors (e.g., a non-targeting control siRNA or an siRNA that targets an unrelated mRNA). The kit also may contain reagents for assessing knockdown of the intended target gene (e.g., primers and probes for quantitative PCR to detect the target mRNA and/or antibodies against the corresponding protein for western blots). Alternatively, the kit may comprise an siRNA sequence or an shRNA sequence and the instructions and materials necessary to generate the siRNA by *in vitro* transcription or to construct an shRNA expression vector.

A pharmaceutical combination in kit form is further provided that includes, in packaged combination, a carrier means adapted to receive a container means in close confinement therewith and a first container means including an interfering RNA composition and an acceptable carrier. Such kits can further include, if desired, one or more of various conventional pharmaceutical kit components, such as, for example, containers with one or more pharmaceutically acceptable carriers, additional containers, etc., as will be readily apparent to those skilled in the art. Printed instructions, either as inserts or as labels, indicating quantities of the components to be administered, guidelines for administration, and/or guidelines for mixing the components, can also be included in the kit.

The ability of interfering RNA to knock-down the levels of endogenous target gene expression in, for example, a human ocular cell line is evaluated *in vitro* as follows. Transformed human cells are plated 24 h prior to transfection in standard growth medium (e.g., DMEM supplemented with 10% fetal bovine serum). Transfection is performed using Dharmafect 1 (Dharmacon, Lafayette, CO) according to the manufacturer's instructions at interfering RNA concentrations ranging from 0.1 nM - 100 nM. Non-targeting control siRNA and lamin A/C siRNA (Dharmacon) are used as controls. Target mRNA levels are assessed by qPCR 24 h post-transfection using, for example, TAQMAN® forward and reverse primers and a probe set that encompasses the target site (Applied Biosystems, Foster City, CA). Target protein levels may be assessed approximately 72 h post-transfection (actual time dependent on protein turnover rate) by western blot, for example. Standard techniques for RNA and/or protein isolation from cultured cells are well-known to those skilled in the art. To reduce the chance of non-specific, off-target effects, the lowest possible concentration of interfering RNA is used that produces the desired level of knock-down in target gene expression.

The ability of interfering RNAs of the present invention to knock-down levels of IGF-IR protein expression is further exemplified in Example 1 as follows.

Accordingly, disclosed herein is at least:

A method for attenuating expression of IGF1R mRNA in an expression system, said method comprising: administering to said expression system a composition comprising an effective amount of interfering RNA having a length of 19 to 49 nucleotides and a pharmaceutically acceptable carrier, said interfering RNA comprising:a sense nucleotide strand, an antisense nucleotide strand, and a region of at least near-perfect contiguous complementarity of at least 19 nucleotides;wherein said antisense nucleotide strand hybridizes under physiological conditions to a portion of mRNA corresponding to SEQ ID NO:1 and has a region of at least near-perfect contiguous complementarity of at least 19 nucleotides with said hybridizing portion of mRNA corresponding to SEQ ID NO:1,wherein said expression of IGF1R mRNA is attenuated thereby.

Use of a medicament for treating ocular angiogenesis in a subject in need thereof, said method comprising: administering to an eye of said subject a composition comprising an effective amount of interfering RNA having a length of 19 to 49 nucleotides and a pharmaceutically acceptable carrier, said interfering RNA comprising: a sense nucleotide strand, an antisense nucleotide strand, and a region of at least near-perfect contiguous complementarity of at least 19 nucleotides; wherein said antisense nucleotide strand hybridizes under physiological conditions to a portion of mRNA corresponding to SEQ ID NO:1, and has a region of at least near-perfect contiguous complementarity of at least 19 nucleotides with said hybridizing portion of mRNA corresponding to SEQ ID NO:1, wherein said ocular angiogenesis is treated thereby.

A method of attenuating expression of IGF1R mRNA in an expression system, said method comprising: administering to said expression system a composition comprising an effective amount of single-stranded interfering RNA having a length of 19 to 49 nucleotides and a pharmaceutically acceptable carrier, wherein said single-stranded interfering RNA hybridizes under physiological conditions to a portion of mRNA corresponding to SEQ ID NO:1 comprising nucleotide 401, 635, 1062, 1548, 1604, 1643, 1766, 1922, 2012, 2069, 2210, 2416, 2423, 2654, 2909, 3339, 3416, 3464, 3476, 3505, 3512, 3781, 3782, 3881, 4064, 4158, 4411, 4487, 4904, 4905, 4909, 3329, 2323 or 2887, and said interfering RNA has a region of at least near-perfect contiguous complementarity of at least 19 nucleotides with said hybridizing portion of mRNA corresponding to SEQ ID NO:1, wherein said expression of IGF1R mRNA is thereby attenuated.

A method of attenuating expression of IGF1R mRNA in an expression systemt, said method comprising: administering to said expression system a composition comprising an effective amount of interfering RNA having a length of 19 to 49 nucleotides and a pharmaceutically acceptable carrier, said interfering RNA comprising: a region of at least 13 contiguous nucleotides having at least 80% sequence complementarity to, or at least 80% sequence identity with, said penultimate 13, 14, 15, 16, 17, or 18 nucleotides of said 3' end of an mRNA corresponding to any one of SEQ ID NO:2, and SEQ ID NO:8 - SEQ ID NO:40, wherein said expression of IGF1R mRNA is attenuated thereby.

The use, in the preparation of a medicament for the treatment of ocular angiogenesis in a subject, of a composition comprising: an effective amount of interfering RNA having a length of 19 to 49 nucleotides and a pharmaceutically acceptable carrier, said interfering RNA comprising: a region of at least 13 contiguous nucleotides having at least 90% sequence complementarity to, or at least 90% sequence identity with, said penultimate 13 nucleotides of said 3' end of an mRNA corresponding to any one of SEQ ID NO:2, and SEQ ID NO:8 - SEQ ID NO:40, wherein said ocular angiogenesis is treated thereby.

The use, in the preparation of a medicament for the treatment of ocular angiogenesis in a subject, of a composition comprising:a double stranded siRNA molecule that down regulates expression of an IGF1R gene via RNA interference, wherein: each strand of said siRNA molecule is independently about 19 to about 27 nucleotides in length; and one strand of said siRNA molecule comprises a nucleotide sequence having substantial complementarity to an mRNA corresponding to said IGF1R gene, respectively, so that said siRNA molecule directs cleavage of said mRNA via RNA interference.

A composition comprising an interfering RNA having a length of 19 to 49 nucleotides and comprising a nucleotide sequence corresponding to any one of SEQ ID NO:2, and SEQ ID NO:8 - SEQ ID NO:40, or a complement thereof, and a pharmaceutically acceptable carrier.

A composition comprising a double stranded siRNA molecule that down regulates expression of an IGF1R gene via RNA interference, wherein: each strand of said siRNA molecule is independently about 19 to about 27 nucleotides in length; and one strand of said siRNA molecule comprises a nucleotide sequence having substantial complementarity to an mRNA corresponding to said IGF1R gene so that said siRNA molecule directs cleavage of said mRNA via RNA interference.

and

A method of attenuating expression of IGF1R mRNA to a subject expressing IGF1R mRNA, said method comprising:administering to said subject a composition comprising an effective amount of interfering RNA having a length of 19 to 49 nucleotides and a pharmaceutically acceptable carrier, said interfering RNA comprising: a sense nucleotide strand, an antisense nucleotide strand, and a region of at least near-perfect contiguous complementarity of at least 19 nucleotides; wherein said antisense nucleotide strand hybridizes under physiological conditions to a portion of mRNA corresponding to SEQ ID NO:1 and has a region of at least near-perfect contiguous complementarity of at least 19 nucleotides with said hybridizing portion of mRNA corresponding to SEQ ID NO:1, wherein said expression of IGFIR mRNA is attenuated thereby.

The invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes to the claims that come within the meaning and range of equivalency of the claims are to be embraced within their scope. Further, all published documents, patents, and applications mentioned herein are hereby incorporated by reference, as if presented in their entirety.

### Example 1

### Interfering RNA for Specifically Silencing IGF1R

The present study examines the ability of IGF1R-interfering RNA to knock down the levels of endogenous IGF-1R protein expression in cultured HeLa cells.

Transfection of HeLa cells was accomplished using standard *in vitro* concentrations (0.1 - 10 nM) of IGF1R siRNAs, siCONTROL RISC-free siRNA #1, or siCONTROL Non-targeting siRNA #2 (NTC2) and DHARMAFECT® #1 transfection reagent (Dharmacon, Lafayette, CO). All siRNAs were dissolved in 1X siRNA buffer, an aqueous solution of 20 mM KCl, 6 mM HEPES (pH 7.5), 0.2 mM MgCl₂. Control samples included a buffer control in which the volume of siRNA was replaced with an equal volume of 1X siRNA buffer (-siRNA). Western blots using an anti-IGF-1Rβ antibody were performed to assess IGF-1R protein expression. This antibody recognizes both the 200-kDa IGF-1R precursor and 97-kDa mature IGF-1Rβ proteins. The IGF1R siRNAs are double-stranded interfering RNAs having specificity for the following targets: siIGF1R #6 targets SEQ ID NO:38; siIGF1R #8 targets SEQ ID NO:39; siIGF1R #17 targets SEQ ID NO:13; siIGF1R #18 targets SEQ ID NO:40. As shown by the data of the figure, the siIGF1R #8 and siIGFIR #17 siRNAs reduced IGF-1R protein expression significantly at the 10 nM and 1 nM concentrations relative to the control siRNAs, indicating that these IGFIR siRNAs are more effective than siIGFIR #6 and siIGFIR #18. None of the siRNAs reduced TGF-1R protein expression significantly at 0.1 nM.

The references cited herein, to the extent that they provide exemplary procedural or other details supplementary to those set forth herein, are specifically incorporated by reference.

Those of skill in the art, in light of the present disclosure, will appreciate that obvious modifications of the embodiments disclosed herein can be made without departing from the spirit and scope of the invention. All of the embodiments disclosed herein can be made and executed without undue experimentation in light of the present disclosure. The full scope of the invention is set out in the disclosure and equivalent embodiments thereof. The specification should not be construed to unduly narrow the full scope of protection to which the present invention is entitled.

As used herein and unless otherwise indicated, the terms "a" and "an" are taken to mean "one", "at least one" or "one or more".

### CLAIMS OF THE PARENT APPLICATION

1. A method for attenuating expression of IGF1R mRNA in an expression system, said method comprising:
   administering to said expression system a composition comprising an effective amount of interfering RNA having a length of 19 to 49 nucleotides and a pharmaceutically acceptable carrier, said interfering RNA comprising:
      a sense nucleotide strand, an antisense nucleotide strand, and a region of at least near-perfect contiguous complementarity of at least 19 nucleotides;
      wherein said antisense nucleotide strand hybridizes under physiological conditions to a portion of mRNA corresponding to SEQ ID NO:1 and has a region of at least near-perfect contiguous complementarity of at least 19 nucleotides with said hybridizing portion of mRNA corresponding to SEQ ID NO:1,
   wherein said expression of IGF1R mRNA is attenuated thereby.
2. Use of a medicament for treating ocular angiogenesis in a subject in need thereof, said method comprising:
   administering to an eye of said subject a composition comprising an effective amount of interfering RNA having a length of 19 to 49 nucleotides and a pharmaceutically acceptable carrier, said interfering RNA comprising:
      a sense nucleotide strand, an antisense nucleotide strand, and a region of at least near-perfect contiguous complementarity of at least 19 nucleotides;
      wherein said antisense nucleotide strand hybridizes under physiological conditions to a portion of mRNA corresponding to SEQ ID NO:1, and has a region of at least near-perfect contiguous complementarity of at least 19 nucleotides with said hybridizing portion of mRNA corresponding to SEQ ID NO: 1,
   wherein said ocular angiogenesis is treated thereby.
3. A method of attenuating expression of IGF1R mRNA in an expression system, said method comprising:
   administering to said expression system a composition comprising an effective amount of single-stranded interfering RNA having a length of 19 to 49 nucleotides and a pharmaceutically acceptable carrier,
      wherein said single-stranded interfering RNA hybridizes under physiological conditions to a portion of mRNA corresponding to SEQ ID NO: comprising nucleotide 401, 635, 1062, 1548, 1604, 1643, 1766, 1922, 2012, 2069, 2210, 2416, 2423, 2654, 2909, 3339, 3416, 3464, 3476, 3505, 3512, 3781, 3782, 3881, 4064, 4158, 4411, 4487, 4904, 4905, 4909, 3329, 2323 or 2887, and said interfering RNA has a region of at least near-perfect contiguous complementarity of at least 19 nucleotides with said hybridizing portion of mRNA corresponding to SEQ ID NO:1,
   wherein said expression of IGF1R mRNA is thereby attenuated.
4. A method of attenuating expression of IGF1R mRNA in an expression systemt, said method comprising:
   administering to said expression system a composition comprising an effective amount of interfering RNA having a length of 19 to 49 nucleotides and a pharmaceutically acceptable carrier, said interfering RNA comprising:
      a region of at least 13 contiguous nucleotides having at least 80% sequence complementarity to, or at least 80% sequence identity with, said penultimate 13, 14, 15, 16, 17, or 18 nucleotides of said 3' end of an mRNA corresponding to any one of SEQ ID NO:2, and SEQ ID NO:8 - SEQ ID NO:40,
      wherein said expression of IGF1R mRNA is attenuated thereby.
5. The use, in the preparation of a medicament for the treatment of ocular angiogenesis in a subject, of a composition comprising: an effective amount of interfering RNA having a length of 19 to 49 nucleotides and a pharmaceutically acceptable carrier, said interfering RNA comprising:
   a region of at least 13 contiguous nucleotides having at least 90% sequence complementarity to, or at least 90% sequence identity with, said penultimate 13 nucleotides of said 3' end of an mRNA corresponding to any one of SEQ ID NO:2, and SEQ ID NO:8 - SEQ ID NO:40,
   wherein said ocular angiogenesis is treated thereby.
6. The use, in the preparation of a medicament for the treatment of ocular angiogenesis in a subject, of a composition comprising::
   a double stranded siRNA molecule that down regulates expression of an IGFIR gene via RNA interference,
   wherein:
      each strand of said siRNA molecule is independently about 19 to about 27 nucleotides in length; and
      one strand of said siRNA molecule comprises a nucleotide sequence having substantial complementarity to an mRNA corresponding to said IGF1R gene, respectively, so that said siRNA molecule directs cleavage of said mRNA via RNA interference.
7. The method of any of Claims 1, 3, or 4, further comprising administering to said expression system a second interfering RNA having a length of 19 to 49 nucleotides, and comprising:
   a sense nucleotide strand, an antisense nucleotide strand, and a region of at least near-perfect complementarity of at least 19 nucleotides;
   wherein said antisense nucleotide strand of said second interfering RNA hybridizes under physiological conditions to a second portion of mRNA corresponding to SEQ ID NO:1 and said antisense strand has a region of at least near-perfect contiguous complementarity of at least 19 nucleotides with said second hybridizing portion of mRNA corresponding to SEQ ID NO:1.
8. A composition comprising an interfering RNA having a length of 19 to 49 nucleotides and comprising a nucleotide sequence corresponding to any one of SEQ ID NO:2, and SEQ ID NO:8 - SEQ ID NO:40, or a complement thereof, and a pharmaceutically acceptable carrier.
9. A composition comprising a double stranded siRNA molecule that down regulates expression of an IGF1R gene via RNA interference, wherein:
   each strand of said siRNA molecule is independently about 19 to about 27 nucleotides in length; and
   one strand of said siRNA molecule comprises a nucleotide sequence having substantial complementarity to an mRNA corresponding to said IGF1R gene so that said siRNA molecule directs cleavage of said mRNA via RNA interference.
10. A method of attenuating expression of IGF1R mRNA to a subject expressing IGFIR mRNA, said method comprising:
   administering to said subject a composition comprising an effective amount of interfering RNA having a length of 19 to 49 nucleotides and a pharmaceutically acceptable carrier, said interfering RNA comprising:
      a sense nucleotide strand, an antisense nucleotide strand, and a region of at least near-perfect contiguous complementarity of at least 19 nucleotides;
      wherein said antisense nucleotide strand hybridizes under physiological conditions to a portion of mRNA corresponding to SEQ ID NO:1 and has a region of at least near-perfect contiguous complementarity of at least 19 nucleotides with said hybridizing portion of mRNA corresponding to SEQ ID NO:1,
   wherein said expression of IGF1R mRNA is attenuated thereby.
11. The method of any of Claims 1, 3, 4, or 10, wherein said antisense strand is designed to target an mRNA corresponding to SEQ ID NO:1 comprising nucleotide 401, 635, 1062, 1548, 1604, 1643, 1766, 1922, 2012, 2069, 2210, 2416, 2423, or 2654.
12. The method of any of Claims 1, 3, 4, or 10, wherein said antisense strand is designed to target an mRNA corresponding to SEQ ID NO:1 comprising nucleotide 2909, 3339, 3416, 3464, 3476, 3505, 3512, 3781, 3782, 3881, 4064, 4158, 4411, 4487, 4904, 4905, 4909, 3329, 2323 or 2887.
13. The method of Claim 1, 3, 4, or 10 or the composition of claim 8 or 9, wherein said composition further comprises a second interfering RNA having a length of 19 to 49 nucleotides and comprising a region of at least 13, 14, 15, 16, 17, or 18 contiguous nucleotides having at least 80% complementarity to, or at least 80% sequence identity with, said penultimate 13 nucleotides of said 3' end of a second mRNA corresponding to any one of SEQ ID NO:2, and SEQ ID NO:8 - SEQ ID NO:40.

## Claims

1. A pharmaceutical composition comprising:
(a) an effective amount of interfering RNA having a length of 19 to 49 nucleotides and a pharmaceutically acceptable carrier, said interfering RNA comprising: a region of at least 13 contiguous nucleotides having at least 90% sequence complementarity to, or at least 90% sequence identity with the penultimate 13 nucleotides of the 3' end of an mRNA corresponding to the DNA sequence of any one of SEQ ID NO:2, and SEQ ID NO:8 - SEQ ID NO:40; and
(b) a pharmaceutically acceptable carrier.

2. A pharmaceutical composition according to claim 1, comprising a double stranded siRNA molecule that down regulates expression of an IGF1R gene via RNA interference, wherein:
each strand of said siRNA molecule is independently about 19 to about 27 nucleotides in length; and
one strand of said siRNA molecule comprises a nucleotide sequence having substantial complementarity to a nucleotide sequence corresponding to the DNA sequence of any one of SEQ ID NO:2, and SEQ ID NO:8 - SEQ ID NO:40, so that said siRNA molecule directs cleavage of said mRNA via RNA interference.

3. A pharmaceutical composition according to claim 1 comprising an interfering RNA having a length of 19 to 49 nucleotides and comprising a nucleotide sequence corresponding to the DNA sequence of any one of SEQ ID NO:2, and SEQ ID NO:8 - SEQ ID NO:40, or a complement thereof, and a pharmaceutically acceptable carrier.

4. The pharmaceutical composition of Claim 3, further comprising a second interfering RNA having a length of 19 to 49 nucleotides and comprising a region of at least 13, 14, 15, 16, 17, or 18 contiguous nucleotides having at least 80% complementarity to, or at least 80% sequence identity with the penultimate 13 nucleotides of the 3' end of a second mRNA corresponding to the DNA sequence of any one of SEQ ID NO:2, and SEQ ID NO:8 - SEQ ID NO:40.

5. A pharmaceutical composition according to claim 1 of interfering RNA having a length of 19 to 49 nucleotides, comprising:
a sense nucleotide strand, an antisense nucleotide strand, and a region of at least near-perfect contiguous complementarity of at least 19 nucleotides;
wherein said antisense nucleotide strand has at least 90% complementarity to a portion of mRNA corresponding to an IGFIR target sequence that comprises nucleotide 401, 635, 1062, 1548, 1604, 1643, 1766, 1922, 2012, 2069, 2210, 2416, 2423, 2654, 2909, 3339, 3416, 3464, 3476, 3505, 3512, 3781, 3782, 3881, 4064, 4158, 4411, 4487, 4904, 4905, 4909, 3329, 2323 or 2887 of SEQ ID NO:1 and has a region of at least near-perfect contiguous complementarity of at least 19 nucleotides with said hybridizing portion of mRNA corresponding to SEQ ID NO:1.

6. A pharmaceutical composition according to Claim 5, further comprising the use of a second interfering RNA having a length of 19 to 49 nucleotides and comprising a region of at least 13, 14, 15, 16, 17, or 18 contiguous nucleotides having at least 80% complementarity to, or at least 80% sequence identity with the penultimate 13 nucleotides of the 3' end of a second mRNA corresponding to the DNA sequence of any one of SEQ ID NO:2, and SEQ ID NO:8 - SEQ ID NO:40.

7. A composition comprising an interfering RNA having a length of 19 to 49 nucleotides and comprising a nucleotide sequence corresponding to any one of SEQ ID NO:2, and SEQ ID NO:8 - SEQ ID NO:40, or a complement thereof, and a pharmaceutically acceptable carrier.

8. A composition comprising a double stranded siRNA molecule that down regulates expression of an IGF1R gene via RNA interference, wherein:
each strand of said siRNA molecule is independently about 19 to about 27 nucleotides in length; and
one strand of said siRNA molecule comprises a nucleotide sequence having substantial complementarity to an mRNA corresponding to said IGF1R gene so that said siRNA molecule directs cleavage of said mRNA via RNA interference.
